Europäisches Patentamt

European Patent Office

Office européen des brevets

(11) Veröffentlichungsnummer: **0 149 464 B1**

# EUROPÄISCHE PATENTSCHRIFT

(45) Veröffentlichungstag der Patentschrift: **17.04.91**

(51) Int. Cl.⁵: **C07J 7/00**, C07J 9/00, A61K 31/57, A61K 31/575

(21) Anmeldenummer: **85100158.6**

(22) Anmeldetag: **09.01.85**

Die Akte enthält technische Angaben, die nach dem Eingang der Anmeldung eingereicht wurden und die nicht in dieser Patentschrift enthalten sind.

(54) **6-Alpha,16-beta-Dimethylkortikoide.**

(30) Priorität: **16.01.84 DE 3401680**

(43) Veröffentlichungstag der Anmeldung:
**24.07.85 Patentblatt 85/30**

(45) Bekanntmachung des Hinweises auf die
Patenterteilung:
**17.04.91 Patentblatt 91/16**

(84) Benannte Vertragsstaaten:
**AT BE CH DE FR GB IT LI LU NL SE**

(56) Entgegenhaltungen:
EP-A- 0 100 874
FR-A- 1 467 003
GB-A- 898 292
US-A- 3 054 811
US-A- 3 290 338

(73) Patentinhaber: **SCHERING AKTIENGESELL-
SCHAFT Berlin und Bergkamen
Müllerstrasse 170/178 Postfach 65 03 11
W-1000 Berlin 65(DE)**

(72) Erfinder: **Annen, Klaus, Dr.
Osthofstrasse 80
W-4400 Münster-Albachten(DE)**
Erfinder: **Laurent, Henry, Dr.
Glambecker Weg 21
W-1000 Berlin 28(DE)**
Erfinder: **Hofmeister, Helmut, Dr.
Weislingenstrasse 4
W-1000 Berlin 28(DE)**
Erfinder: **Wiechert, Rudolf, Prof.
Petzower Strasse 8A
W-1000 Berlin 39(DE)**
Erfinder: **Töpert, Michael, Dr.
Sigismundkorso 58
W-1000 Berlin 28(DE)**

## Beschreibung

Die Erfindung betrifft die in den Patentansprüchen gekennzeichneten 6,16-Dimethylkortikoide und pharmazeutische Präparate, die diese Verbindungen enthalten.

Die neuen 6,16-Dimethylkortikoide zeichnen sich gegenüber dem in der GB-A 898292 erwähnten 17α-Acetoxy-11β-hydroxy-6α,16β-dimethyl-1,4-pregnadien-3,20-dion bei topischer Applikation durch eine ausgeprägte antiinflammatorische Wirksamkeit und eine ausgezeichnete Dissoziation zwischen erwünschter topischer Wirksamkeit und unerwünschten systemischen Nebenwirkungen aus.

Die neuen 6,16-Dimethylkortikoide eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die nachfolgenden Ausführungsbeispiele dienen zur Erläuterung der Erfindung.

### Beispiel 1

A) Eine Suspension von 50 g 17α-Hydroxy-16β-methyl-4.9(11)-pregnadien-3.20-dion in 625 ml Diethylglykoldimethylether wird nach Zugabe von 75 g 4-Dimethylaminopyridin mit 75 ml Propionsäureanhydrid 3 Tage bei 80 °C Badtemperatur gerührt. Nach der Eiswasserfällung wird wie üblich aufgearbeitet und das Rohprodukt an 3.5 kg Kieselgel mit einem Methylenchlorid-Aceton-Gradienten (0-12 % Aceton) gereinigt. Ausbeute 34.2 g 16β-Methyl-17α-propionyloxy-4.9(11)-pregnadien-3.20-dion. Schmp. 158-160 °C.

B) Eine Suspension von 50.0 g Natriumacetat in 1500 ml Chloroform und 1500 ml Formaldehyddimethylacetal wird mit 95 ml Phosphoroxychlorid 1 h bei 65 °C Badtemperatur gerührt. Nach Zugabe von 53.0 g 16β-Methyl-4.9(11)-17α-propionyloxy-pregnadien-3.20-dion tropft man weitere 19 ml Phosphoroxychlorid hinzu und rührt 6.5 h bei 65 °C weiter. Die abgekühlte Reaktionslösung wird mit soviel einer gesättigten Sodalösung behandelt, bis die wäßrige Phase alkalisch bleibt. Man trennt die organische Phase ab, wäscht neutral und engt nach dem Trocknen ein. Das Rohprodukt wird an 2500g Kieselgel mit einem Hexan-Essigester-Gradienten (0-50 % Essigester) gereinigt. Man isoliert 33,9 g 16β-Methyl-6-methylen-17α-propionyloxy-4.9(11)-pregandien-3.20-dion. Schmp. 175-177 °C.

C) Eine Suspension von 0.5 g Palladium/Aktivkohle in 30 ml Ethanol und 25 ml Cyclohexen wird 1 h bei 80 °C Badtemperatur gerührt und nach Zugabe von 5,0 g 16β-Methyl-6-methyl-17α-propionyloxy-4.9(11)-pregandien-3,20-dion 16 h unter Rühren erhitzt. Nach dem Abkühlen wird der Katalysator abgesaugt, mit Methylenchlorid gewaschen und die vereinigten Filtrate mit 50 ml konz. Salzsäure 0.5 h bei Raumtemperatur gerührt. Man engt die Reaktionslösung auf 1/3 ihres Volumens ein, gibt auf Eiswasser und arbeitet wie üblich auf. Das Rohprodukt wird an 500 g Kieselgel mit einem Hexan-Essigester-Gradienten (0-50 % Essigester) gereinigt. Ausbeute 3,7 g 6α-16β-Dimethyl-17α-propionyloxy-4.9(11)-pregnadien-3,20-dion. Schmp. 129 °C.

D) Eine Lösung von 3.0 g 6α.16β-Dimethyl-17α-propionyloxy-4.9(11)-pregnadien-3.20-dion in 61 ml Dioxan wird mit 3.0 g Dichlorodicyanobenzochinon 7 h refluxiert. Nach dem Abkühlen und Abfiltrieren engt man das Filtrat zur Trockne ein. Das Rohprodukt wird an 750 g Kieselgel mit einem Hexan-Essigester-Gradienten (0-50 % Essigester) gereinigt. Das erhaltene Rohprodukt wird in 20 ml siedendem Ethanol tropfenweise mit einer Lösung von 5.73 g $Na_2S_2O_5$ in 8 ml Wasser behandelt werden. Nach 2 h wird das Reaktionsgemisch in der Weise destilliert, daß das Volumen in der Destillationsblase durch Wasserzufuhr erhalten bleibt und das Brückenthermometer 99 °C anzeigt. Man kühlt die Destillationsblase auf +20 °C ab, filtriert ab, wäscht den Rückstand gründlich mit Wasser und löst ihn in Methylenchlorid. Die organische Lösung wird nach dem Trocknen über eine Kieselgelschicht filtriert und anschließend eingeengt, und man erhält 2,1 g 6α.16β-Dimethyl-17α-propionyloxy-1,4,9(11)-pregnatrien-3,20-dion. Schmp. 220-221° C.

E ) Eine Lösung von 2,0 g 6α.16β-Dimethyl-17α-propionyloxy-1,4,9(11)-pregnatrien-3.20-dion in 20 ml Dioxan und 2 ml Wasser wird nach Zugabe von 1,1 g N-Bromsuccinimid bei 20° C tropfenweise mit einer Lösung von 0.04 ml 70proz. Perchlorsäure in 1,8 ml Wasser versetzt. Man rührt 1 h bei 20° C Innentemperatur weiter, kühlt auf 15° C herunter und neutralisiert tropfenweise mit einer Lösung aus 3,2 g Natriumacetat und 2,4 g Natriumsulfit in 30 ml Wasser. Dabei darf die Innentemperatur 23° C nicht übersteigen. Nach Zugabe von 5 ml Methanol wird das Reaktionsgemisch 15 min bei Raumtemperatur und nach Zugabe von 20 ml Wasser 3 h bei 0° C weitergerührt. Schließlich saugt man den Niederschlag ab, wäscht den Rückstand mit Wasser neutral und trocknet bei 70° C im Vakuumtrockenschrank. Das Rohprodukt wird aus Aceton/Hexan umkristallisiert. Man isoliert 2,1 g 9α-Brom-11β-hydroxy-6α.16β-dimethyl-17α-propionyloxy-1,4-pregnadien-3.20-dion. Schmp. 175 - 176° C.

F) 500 mg 9α-Brom-11β-hydroxy-6α.16β-dimethyl-17α-propionyloxy-1,4-pregnadien-3,20-dion werden in 10 ml wasserfreiem Tetrahydrofuran gelöst und nach Zugabe von 1.5 ml Tributylzinnhydrid und 40 mg Azobisisobutyronitril 2 h refluxiert. Man engt im Vakuum ein und reinigt den Rückstand an 100 g Kieselgel mit einem Hexan-Essigester-Gradienten (0-50 % Essigester). Ausbeute 270 mg 11β-Hydroxy-6α.16β-dimethyl-17α-propionyloxy-1,4-pregnadien-3,20-dion. Schmp. 260-261° C.

Beispiel 2

A) Eine auf 0° C abgekühlte Suspension von 11.1 g 17α-Hydroxy-16β-methyl-4.9(11)-pregnadien-3.20-dion in 110 ml Buttersäure wird tropfenweise mit 60 ml Trifluoressigsäureanhydrid versetzt und 2 h bei Raumtemperatur gerührt. Nach der Eiswasser-Kochsalz-Fällung wird wie üblich aufgearbeitet und gereinigt. Ausbeute 7.8 g 17α-Butyryloxy-16β-methyl-4.9(11)-pregnadien-3.20-dion. Schmp. 131-132° C.

B) Wie im Beispiel 1B) beschrieben, werden 13.0 g 17α-Butyryloxy-16β-methyl-4.9(11)-pregnadien-3.20-dion mit Formaldehyd-dimethylacetal umgesetzt, aufgearbeitet und gereinigt. Ausbeute 7.8 g 17α-Butyryloxy-16β-methyl-6-methylen-4.9(11)-pregnadien-3.20-dion. Schmp. 133° C.

C) Analog Beispiel 1C) werden 9.2 g 17α-Butyryloxy-16β-methyl-6-methylen-4.9(11)-pregnadien-3.20-dion mit Palladium/A-Kohle und Cyclohexen hydriert, aufgearbeitet und gereinigt. Man isoliert 6.75 g 17α-Butyryloxy-6α.16β-dimethyl-4.9(11)-pregnadien-3.20-dion. Schmp. 165-166° C.

D) Analog Beispiel 1E) werden 4.75 g 17α-Butyryloxy-6α.16β-dimethyl-4.9(11)-pregnadien-3.20-dion mit Dichlorodicyanobenzochinon umgesetzt, aufgearbeitet und gereinigt. Man isoliert 3.3 g 17α-Butyryloxy-6α.16β-dimethyl-1.4.9(11)-pregnatrien-3.20-dion. Schmp. 195-196° C.

E) Wie im Beispiel 1E) beschrieben, werden 3.2 g 17α-Butyryloxy-6α.16β-dimethyl-1.4.9(11)-pregnatrien-3.20-dion mit N-Bromsuccinimid und Perchlorsäure umgesetzt und aufgearbeitet. Man kristallisiert aus Aceton/Hexan um und erhält 2.8 g 9α-Brom-17α-butyryloxy-11β-hydroxy-6α.16β-dimethyl-1.4-pregnadien-3.20-dion. Schmp. 170-171° C.

F) 500 mg 9α-Brom-17α-butyryloxy-11β-hydroxy-6α.16β-dimethyl-1.4-pregnadien-3.20-dion werden analog Beispiel 1F) mit Tributylzinnhydrid debromiert, aufgearbeitet und chromatographiert. Man isoliert 350 mg 17α-Butyryloxy-11β-hydroxy-6α.16β-dimethyl-1.4-pregnadien-3.20-dion. Schmp. 190-191° C.

**Ansprüche**

1. 11β-Hydroxy-6α,16β-dimethyl-17α-propionyloxy-1,4-pregnadien-3,20-dion.

2. 17α-Butyryloxy-11β-hydroxy-6α,16β-dimethyl-1,4-pregnadien-3,20-dion.

3. Pharmazeutische Präparate gekennzeichnet durch einen Gehalt von ein oder zwei 6α,16β-Dimethylkortikoiden gemäß Patentanspruch 1 und 2.

**Claims**

1. 11β-Hydroxy-6α,16β-dimethyl-17α-propionyloxy-1,4-pregnadiene-3,20-dione.

2. 17α-Butyryloxy-11β-hydroxy-6α,16β-dimethyl-1,4-pregnadiene-3,20-dione.

3. Pharmaceutical preparations characterised by a content of one or two 6α,16β-dimethylcorticoids according to patent claims 1 and 2.

**Revendications**

1. Hydroxy-11$\beta$ diméthyl-6$\alpha$,16$\beta$ propionyloxy-17$\alpha$ prégnadiène-1,4 dione-3,20.

2. Butyryloxy-17$\alpha$ hydroxy-11$\beta$ diméthyl-6$\alpha$,16$\beta$ prégnadiène-1,4 dione-3,20.

3. Compositions pharmaceutiques caractérisées en ce qu'elles contiennent un ou deux diméthyl-6$\alpha$,16$\beta$ corticostéroïdes selon l'une des revendications 1 et 2.